# EUROPEAN PATENT APPLICATION

(11) **EP 2 666 446 A1**
(43) Date of publication of application: **27.11.2013**
(21) Application number: 13169187.5
(22) Date of filing: 24.05.2013
(51) Int. Cl.: A61F 13/02

(54) **Contusion tape**

(30) Priority: 24.05.2012 CA 2777975
(71) Applicant: Arbesman, Ray, Toronto, Ontario M5P 1V2 (CA)
(72) Inventor: Arbesman, Ray, Toronto, Ontario M5P 1V2 (CA)
(74) Representative: Bates, Alan Douglas Henry

(57) **Abstract**

A contusion patch comprising a high stretch therapeutic tape with an adhesive backing is disclosed in this specification. The tape has an anchoring portion that adheres to a contusion and a plurality of fingers that extend from the anchoring portion. The tape is less than about 12.7 cm (5 inches) in length and about 5.08 cm (2 inches) in width when unstretched. A frangible release liner covers the adhesive backing of the tape prior to application. The release liner has a greater surface area than the tape, so that an exposed strip of the release liner extends around the tapes perimeter. The release liner is scored at junctions between the anchoring portion and the fingers to allow selective removal during staggered application of the tape.

## Description

### FIELD OF THE INVENTION

The invention relates to articles for use in the treatment of minor soft tissue injuries, and more particularly to an adhesive contusion patch for use in the treatment of contusions and minor skin abrasions.

### BACKGROUND OF THE INVENTION

A relatively new tool in the physiotherapy and sports medicine arsenal is high-stretch adhesive support tape. Such tape is distinct from traditional "sports" tape which is used to isolate and restrain a body part to protect it and allow healing. It is also distinct from traditional Tensor^{®}-type wrap bandages, which although stretchable, are primarily used for bracing an injury. High-stretch adhesive support tape, by contrast, is used for the purpose of positioning a body part (typically a joint) while permitting a high degree of natural mobility and is made from a high-stretch woven fabric matrix with a strong adhesive backing.

High-stretch adhesive support tape is used by physiotherapists and other sports medicine practitioners to support joints, muscles, tendons, ligaments and other parts of the body. The tape is generally used to assist weak musculature by placing a mild degree of tension across the supported body part, in effect acting as an auxiliary muscle. The tension applied by the tape is generally controlled by regulating the amount the tape is stretched during application.

The tape adhesive is strong enough that opposite ends of a length of tape applied to the body will remain adhered even when the tape length is under tension and the body part is in regular active use. This contrasts with other tapes that are either non-adhesive or adhesive only to themselves. These tapes can be wrapped repeatedly on a body part or dispensed from a self-adhesive roll but cannot be adhered directly to the body.

One particularly effective type of high-stretch adhesive support tape is Kinesio Tex™ by Kinesio Co., Ltd. of Japan. This tape has many of the advantageous properties discussed above. However, it suffers several drawbacks, relating to the fact that it is sold in rolls, which must be customized for application according to the imagination and skill of the doctor. A physiotherapist or sports doctor must cut off a section of the tape from a roll, cut the length of tape further into a therapeutic shape, and apply it to the patient. However, cutting the tape inadvertently compromises the tapes underlying fabric weave, creating loose ends in the thread matrix that cause the tape to fray and lift from the skin. Additionally, the cutting, shaping and positioning (tensioning) process are left to the complete discretion of the doctor. It is a trial and error process. The doctor is effectively trying to estimate a position, length, shape and degree of tension that will cause a subjective improvement in sensation, range of motion or pain relief in the patient.

Some manufacturers have introduced precut lengths of tape in several simple shapes to address fraying concerns. These manufacturers also provide instructions for applying the tape to various parts of the body. However, the application process (positioning and tensioning the tape) has not changed and the instructions are often complicated, requiring several precut lengths to be applied in various orientations and tensions. The tape may also still need to be cut before it can be applied in some circumstances, negating the advantage of precut lengths.

Pre-shaped support articles are a recent development that addresses several deficiencies of roll tape and simple precut tape lengths. Pre-shaped support articles are cut into body part specific shapes prior to sale and are often packaged with instructions for their application. Some examples of precut support articles are disclosed in United States patent application 12/526,829 (published as US 2010/0016771).

SpiderTech® precut supports are one particularly effective type of shape tailored high stretch adhesive support tape. Each precut SpiderTech® support article has an specific shape that is targeted at a particular body part. Such shapes may be quite elaborate. Some of the highly customized kinesiology tape supports produced by SpiderTech® are illustrated in the followong US design patents: USD 608,896 - shoulder tape; USD 616,554 - knee tape; USD 625,828 - neck tape; USD 616,553 - lower back tape; USD 335,718 - calf and lower leg tape; USD 613,415 - foot arch tape; USD 608,893 - wrist tape.

Pre-shaped kinesiology tape supports are often accompanied by comprehensive instructions (either included in the support packaging or online) that explain how the tape is to be applied. The instructions typically specify how each section of the tape should be positioned on a specific body part and the amount of tension (stretch) that should be applied to each section. Packaged instructions typically have several diagrams that step the user through the application process. Web based demonstrational videos are also used by some manufacturers and the tape packaging may direct a user to the manufacturer's website for guidance. Demonstrational videos are typically presented by a physician or other health professional who applies the manufacturer's tape to a model in order to exhibit the correct application procedure.

Despite these advances in kinesiology tape technology and education, effective application of the tape is still complicated and may be beyond the capabilities of most end users. In fact, many manufacturers offer educational courses to teach physiotherapists and other health professionals how to apply their particular form of tape.

Further, none of these products is particularly suitable for small and very localized minor injuries (e.g. localized contusions or bruises).

### SUMMARY OF THE INVENTION

It would be desirable to provide a simple patch of versatile precut kinesiology tape that can be used on any small area of the body. For example, it is believed that such a patch could be particularly useful in the treatment of contusions (bruises) or other localized minor injuries or swelling. According to a first aspect of the invention, a contusion patch kit is provided. The kit includes a high stretch therapeutic tape with an adhesive backing (the tape having an anchoring portion that adheres to a contusion and a plurality of fingers that extend from the anchoring portion), a frangible release liner covering the adhesive backing of the tape, and instructions directing a user to apply the contusion patch to a nonspecific part of the user's body using any orientation of the fingers in the user's discretion. The release liner has a greater surface area than the tape so that an exposed strip of the release liner extends around the tape's perimeter. Further, the release liner is scored at junctions between the anchoring portion and the fingers to allow selective removal of the release liner during staggered application of the tape.

The therapeutic tape of the contusion patch is preferably less than about 5 inches in length and about 2 inches in width when unstretched. Ideally the contusion patch is made from therapeutic tape with a stretch ratio that corresponds to the elasticity of human skin. The stretch ratio of the therapeutic tape may be about 1.4 or greater.

The release liner may be marked with visual indicators to direct application of the patch. Specifically, the release liner may be marked with numerals that indicate the order in which sections of the patch are to be applied.

In one embodiment, the tape preferably has a central anchoring portion and four fingers that extend from the anchoring portion, the fingers being arranged in pairs that extend from opposing sides of the anchoring portion to allow the tape to be applied in an 'X' configuration.

In another embodiment, the tape preferably has a central anchoring portion and two fingers that extend from one side of the anchoring portion to allow the tape to be applied in a 'Y' configuration.

The contusion patch may be supplied in a kit with instructions for applying the patch to a contusion. Ideally, the instructions direct a user to:
i. separate the release liner along the scored junction between the anchoring portion and the fingers,
ii. remove the release liner from the anchoring portion of the tape to expose the adhesive backing,
iii. apply the exposed adhesive backing of the anchoring portion to a contusion so that the tape adheres to the user's skin, and
iv. remove the release liner from each of the fingers in turn and apply the exposed adhesive backing to the user's skin.

The instructions preferably direct the user to stretch each finger after removing the release liner and secure the respective finger in place while stretched by pressing the exposed adhesive backing against the user's skin. Preferably the instructions also direct the user to secure the anchoring portion of the tape to the user's skin without any tension in the anchoring portion.

The kit may include a plurality of contusion patches. Ideally the individual patches would be connected end-to-end, with each patch being folded flat and successive patches resting on top of a previous patches. Preferably adjacent patches would be connected by the frangible release liner. The frangible release liner may be scored between adjacent patches to allow the individual patches to be easily separated.

The therapeutic tape may be coated with a dermatologically-acceptable acrylic adhesive to create the adhesive backing.

According to a second aspect of the invention, a contusion patch kit is provided including a high stretch therapeutic tape with an adhesive backing, a frangible release liner covering the adhesive backing of the tape, and instructions for applying the contusion patch to a nonspecific part of the user's body using any orientation of the fingers in the user's discretion. The tape is functionally divided into sections including an anchoring portion that adheres to a contusion and a plurality of fingers that extend from the anchoring portion. The release liner has a greater surface area than the tape so that an exposed strip of the release liner extends around the tape's perimeter. Further, the release liner is scored at junctions between adjacent sections of the tape to allow selective removal of the release liner during staggered application of the tape.

The release liner is marked with indicators that dictate the order in which each section of the tape is to be applied. The instructions reference the release liner indicators and direct a user to:
i. separate the release liner along the scored junction between adjacent sections of the tape,
ii. remove the release liner from a particular section of the tape indentified by one of the indicators to expose the adhesive backing of that section,
iii. apply the exposed adhesive backing of the section to a contusion so that the tape adheres to the user's skin, and
iv. remove the release liner from each of the other sections in an order dictated by the indicators marked on the release liner in turn and apply the exposed adhesive backing to the user's skin.

The instructions preferably direct the user to stretch each finger after removing the release liner and secure the respective finger in place while stretched by pressing the exposed adhesive backing against the user's skin. Preferably the instructions also direct the user to secure the anchoring portion of the tape to the user's skin without any tension in the anchoring portion.

The release liner preferably has a greater surface area than the tape so that an exposed strip of the release liner extends around the tapes perimeter to reduce accidental exposure of the adhesive backing (and prevent fraying of the edges prior to use).

The therapeutic tape of the contusion patch is preferably less than about 5 inches in length and about 2 inches in width when unstretched. Ideally, the contusion patch is made from therapeutic tape with a stretch ratio that corresponds to the elasticity of human skin. The stretch ratio of the therapeutic tape may be about 1.4 or greater.

The release liner may be marked with visual indicators to direct application of the patch. Specifically, the release liner may be marked with numerals that indicate the order in which sections of the patch are to be applied.

In one embodiment, the tape preferably has a central anchoring portion and four fingers that extend from the anchoring portion, the fingers being arranged in pairs that extend from opposing sides of the anchoring portion to allow the tape to be applied in an 'X' configuration.

In another embodiment, the tape preferably has a central anchoring portion and two fingers that extend from one side of the anchoring portion to allow the tape to be applied in a 'Y' configuration.

The kit may include a plurality of contusion patches. The individual patches may be connected end-to-end, with each patch being folded flat and successive patches resting on top of a previous patches. Preferably adjacent patches would be connected by the frangible release liner. The frangible release liner may be scored between adjacent patches to allow the individual patches to be easily separated.

The therapeutic tape may be coated with a dermatologically-acceptable acrylic adhesive to create the adhesive backing.

The contusion patches disclosed in this specification are defined in some circumstances by their 'stretch ratio'. For the purpose of this specification, the 'stretch ratio' represents the percentage elongation of the underlying tape under tension and is a measure of the maximum or final stretched length divided by the initial unstretched length (λ = I / L).

### BRIEF DESCRIPTION OF THE FIGURES

Figure 1 is a top elevation of an 'X' configuration contusion patch after application to a contusion.
Figure 2 is a top elevation of the contusion patch illustrated in Figure 1 prior to application with a removable release liner covering the adhesive backing.
Figure 3 is a bottom elevation of the contusion patch illustrated in Figure 1 depicting numerical indications printed on the various sections of the release liner and scored delimitations between adjacent sections of the contusion patch.
Figure 4 is a top elevation of an 'Y' configuration contusion patch after application to a contusion.
Figure 5 is a top elevation of the contusion patch illustrated in Figure 4 prior to application with a removable release liner covering the adhesive backing.
Figure 6 is a bottom elevation of the contusion patch illustrated in Figure 4 depicting numerical indications printed on the various sections of the release liner and scored delimitations between adjacent sections of the contusion patch.
Figure 7 is a top schematic representation of the contusion patch of Figure 4 demonstrating the stretch ratio of the therapeutic tape. The anchoring portion and one of the fingers have been applied to a subject while another finger is unstretched and is still attached to the release liner.
Figure 8 is a side elevation of a plurality of contusion patches connected end-to-end and stacked flat in a pile.
Figure 9 is a flow chart representation of a method for applying a contusion patch.

### DETAILED DESCRIPTION

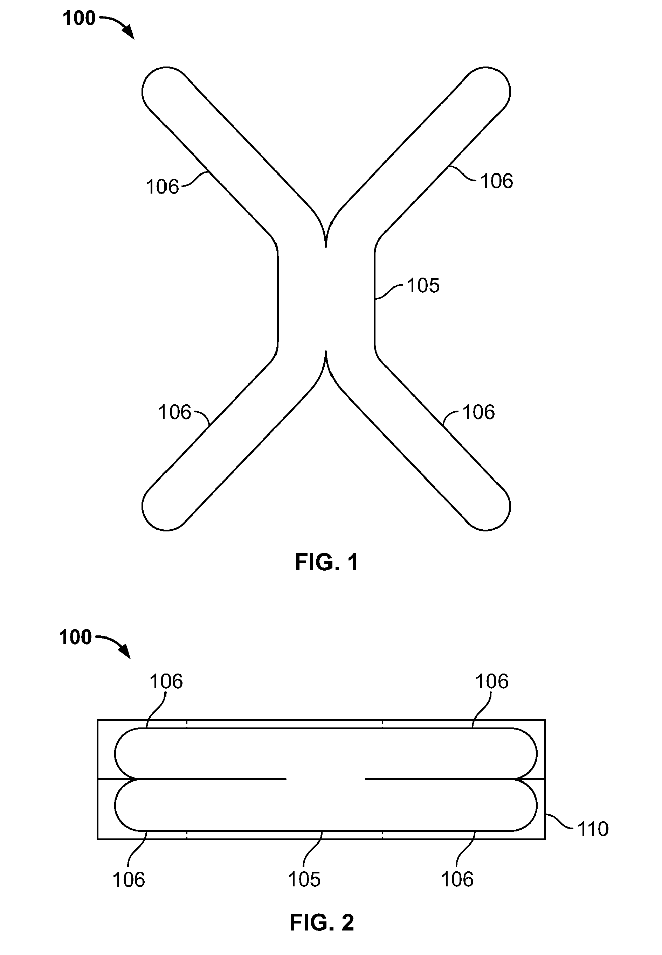

Two embodiments of contusion patch are illustrated in Figures 1 to 8. The patches **100, 200** are for use in the treatment of soft tissue injuries such as contusions and minor abrasions. Each patch **100, 200** is easily applied to any minor soft tissue injury irrespective of which body part has sustained the injury. Furthermore, the orientation of the patch **100, 200** on the user's body does not influence its effectiveness, as the patch is not compensating for a musculature imbalance or strain. This universal applicability and uncomplicated application process make the patch **100, 200** suitable for general consumer use.

Each patch **100, 200** is fabricated from a high stretch therapeutic tape **101** with an adhesive backing. The adhesive backing secures the tape **101** to a contusion when the patch **100, 200** is applied to a user's skin. In both illustrated embodiments 100, 200, the tape **101** is less than about 5 inches (13cm) in length and about 2 inches (5cm) in width when unstretched. This compact size allows the patch to be used on small injuries (e.g. localized bruises), and gives less opportunity for the exposed tape to stick to itself. This contrasts with more elaborate tapes currently on the market.

The tape **101** is functionally divided into sections. The sections are preferably applied to a user's skin in a prescribed sequence. Each section is made from the same strip of material and shares the same adhesive backing. The functional sections of the illustrated tape **101** include an anchoring portion **105** and a plurality of fingers **106** that extend from the anchoring portion **105.** The anchoring portion **105** of the tape **101** is adhesively secured to the skin directly over a contusion. The fingers **106** are adhesively secured to surrounding areas of the user's skin without regard to their orientation on the user's body. The adhesive backing extends uninterrupted across each section of the tape **101.**

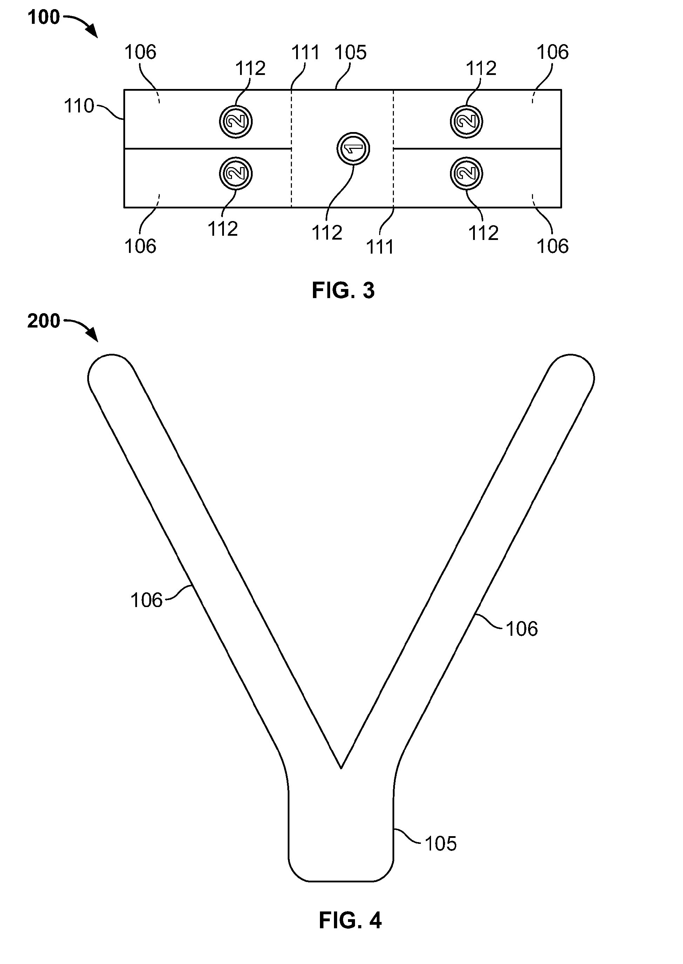

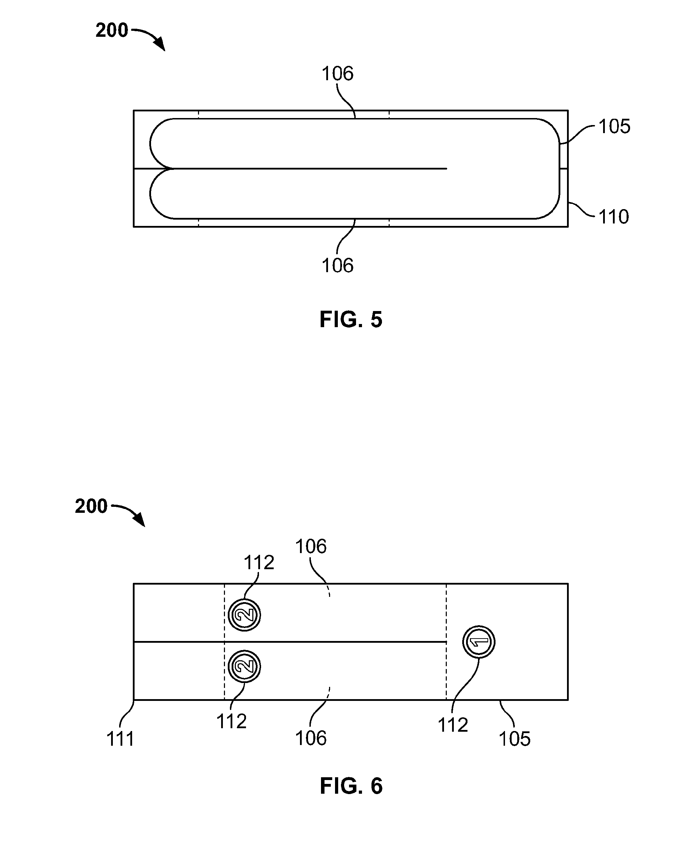

The adhesive backing of the tape **101** is covered with a frangible release liner **110.** The release liner **110** is removed before the tape **101** is applied. The release liner **110** reduces contamination of the adhesive backing (preventing dust and other debris from sticking to the adhesive) and inadvertent adhesion during handling. The release liner **110** is scored at the junctions between adjacent sections of the tape **101** (as illustrated in Figure 3 and 6). The illustrated release liner **110** has scoring **111** between the anchoring portion **105** and the fingers **106.** The scoring **111** assists separation of the release liner **110** covering adjacent sections of the tape **101,** allowing portions of the release liner **110** to be selectively removed during application. Ideally the release liner **110** is only removed from a section of the tape **101** when the user is ready to apply the respective section. This prevents the adhesive backing from inadvertently sticking to the user or their clothing.

The release liner **101** has a greater surface area than the corresponding tape **101.** A strip **115** of exposed release liner **110** extends beyond the parameter of the tape 101 in the illustrated embodiments. The exposed strip **115** extends around the entire perimeter of the tape **101,** creating a boarder that resists edges of the tape **101** inadvertently peeling away from the release liner **110.**

The contusion patches may be marked with visual indicators to direct a user when applying the patch **100, 200** to an injury. The markings are ideally applied to the release liner **110.** The illustrated contusion patches **100, 200** have numerals **112** printed on the respective release liners **111.** The numerals **112** indicate the staggered order in which each section of the tape **101** is to be applied.

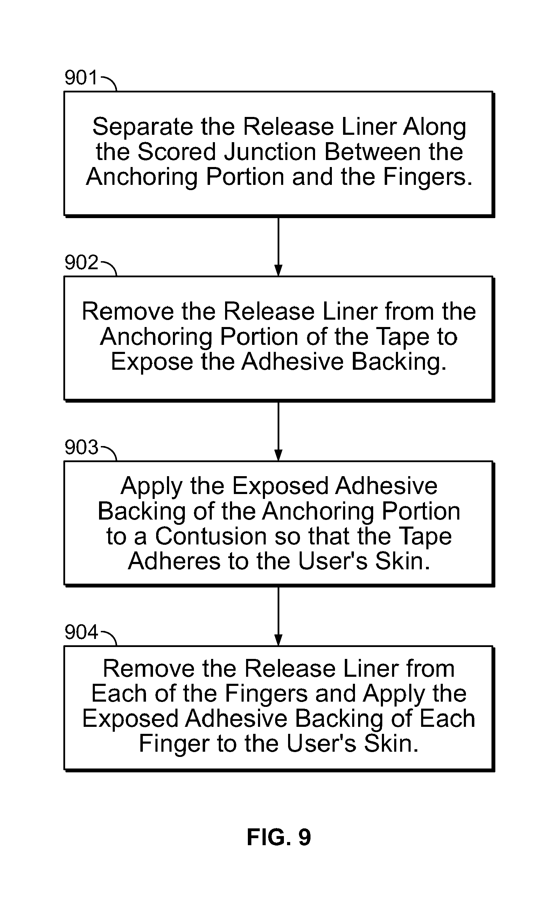

The patches **100, 200** may also be supplied with instructions that assist the user to apply the tape **101.** The instructions ideally direct the user to apply the patch **100, 200** to a nonspecific part of the user's body without regard to the orientation of the fingers and may reference visual indicators marked on the tape **101** or release liner **100, 200.** An exemplary set of instructions for applying the contusion patch to a user's skin are presented in Figure 9. The instructions direct the user to:
i. separate the release liner **110** along the scored junction **111** between adjacent sections of the tape **101,** such as the junction between the anchoring portion **105** and the fingers **106** (step **901),**
ii. remove the release liner **110** from a particular section of the tape **101** to expose the adhesive backing of that section, ideally sections of the release liner **110** are removed in an order identified by marked indicators and the release liner **110** is removed from the anchoring portion **105** of the tape **101** first (step **902),**
iii. apply the exposed adhesive backing of the first section (ideally the anchoring portion **105)** to a contusion so that the tape **101** adheres to the user's skin (step **903),** and
iv. remove the release liner **110** from each of the remaining sections (usually the fingers **106)** and apply the exposed adhesive backing of each section/finger **106** in turn to the user's skin (step **904).**

The instructions may also direct the user to tension sections of the tape **101** during application. The instructions may direct the user to stretch one or more of the fingers **106** after the release liner **110** has been removed and to secure the respective finger **106** to the user's skin while stretched. This retains some tension in finger **106** following adhesion to the user's skin. The residual tension pulls the anchoring portion 105 toward the finger **106,** lifting the skin over the contusion. The user may be instructed to secure the anchoring portion **105** of the tape **101** without stretching, so that there is no pretensioning in the anchoring portion **105.**

Each section of the tape **101** is secured in place by pressing the exposed adhesive backing against the user's skin. Ideally, the release liner **110** is only removed from each section when the user is ready to apply the respective section. For instance, the anchoring portion **105** may be secured to the contusion before the release liner **110** is removed from any of the fingers **106.** The release liner **110** is ideally removed from each finger **106** in turn, so that the adhesive backing is only exposed immediately prior to application.

The tape **101** is preferably breathable. The illustrated tape **101** has a woven fabric base that allows liquid to pass through gaps between adjacent threads in the weave. One side of the fabric is coated with a skin-safe high tack adhesive (the adhesive backing) that holds the tape **101** in contact with the skin. The coating is ideally a high strength dermatologically-acceptable acrylic adhesive.

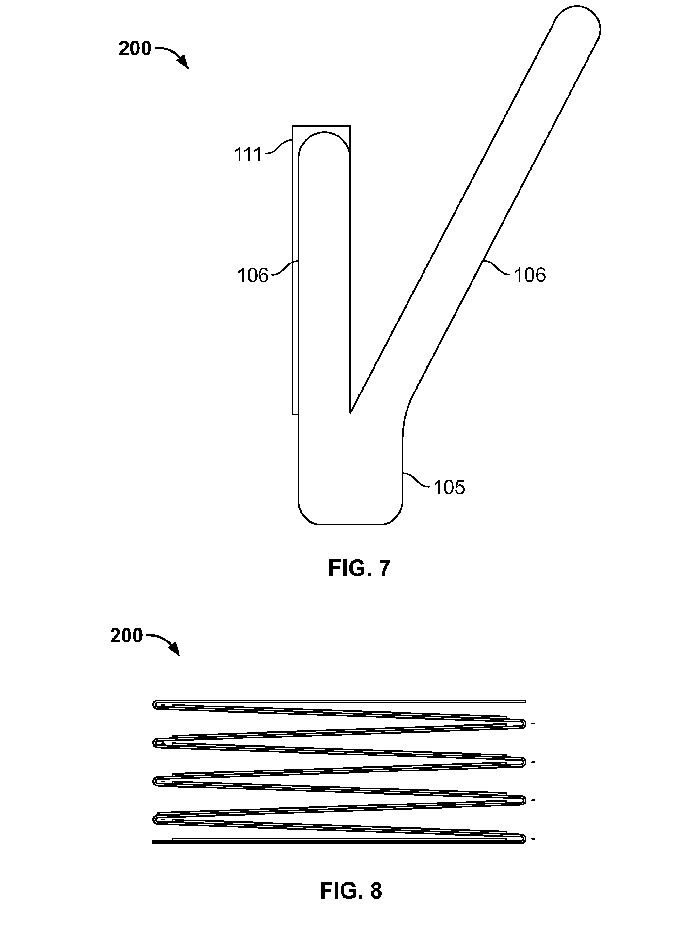

The underlying fabric weave of the tape **101** produces desirable anisotropic deformation characteristics when the tape **101** is stretched. The tape **101** exhibits a high stretch ratio when tensioned coaxially with the fingers **106** (as illustrated in Figure 8). Ideally the stretch ratio of the tape **101** when extended coaxially with the fingers **106** roughly equates to the elasticity of human skin. This corresponds to a stretch ratio of about 1.4. The stretch ratio may be higher without adversely affecting the tapes performance. The tape **101** is significantly more resistant to deformation transverse to the direction the fingers **106** extend. This resistance to transverse deformation maintains the tape **101** width when the fingers **106** are stretched, preventing significant inward contraction. It also opposes transverse extension of the tape if tension is applied transverse to the fingers **101.**

An 'X' configuration patch **100** is illustrated in Figures 1 to 3. The 'X' configuration patch **100** has four fingers **106** that extend from the central anchoring portion **105.** The fingers **106** are arranged in pairs that extend from opposing sides of the anchoring portion **105.** An alternate 'Y' configuration patch **200** is illustrated in Figures 4 to 6. The 'Y' configuration patch **200** has two fingers **106** that both extend from the same side of the anchoring portion **105.** Both tape configurations can be applied without concern for orientation so long as the anchoring portion is secured to the skin over the contusion. The illustrated contusion patches may be bundled together in packs or kits containing multiple patches. The patches are ideally connected end-to-end and stacked flat within the desired packaging to reduce the overall size of the kit. This stacking arrangement is illustrated in Figure 8. Adjacent patches in the kit are connected by the frangible release **110** liner. The patches are folded one atop the other, with each successive patch resting on top of a previous patch as illustrated. The frangible release liner is scored between adjacent patches (similarly to the scoring between adjacent sections of each patch) in the kit to facilitate separation of adjacent patches.

## Claims

1. A contusion patch kit comprising:
a high stretch therapeutic tape with an adhesive backing, the tape having an anchoring portion that adheres to a contusion and a plurality of fingers that extend from the anchoring portion,
a frangible release liner covering the adhesive backing of the tape, the release liner having a greater surface area than the tape so that an exposed strip of the release liner extends around the tape's perimeter, the release liner being scored at junctions between the anchoring portion and the fingers to allow selective removal of the release liner during staggered application of the tape, and
instructions directing a user to apply the contusion patch to a nonspecific part of the user's body using any orientation of the fingers in the user's discretion.

2. The kit of claim 1 wherein the therapeutic tape is less than about 5 inches in length and about 2 inches in width when unstretched.

3. The kit of claim 1 or claim 2 wherein the therapeutic tape has a stretch ratio of about 1.4 or greater.

4. The kit of claims 1, 2, or 3 wherein the release liner is marked with numerals that indicate the order in which sections of the patch are to be applied.

5. The kit of any preceding claim wherein the tape has four fingers that extend from the anchoring portion, the fingers being arranged in pairs that extend from opposing sides of the anchoring portion to allow the tape to be applied in an 'X' configuration.

6. The kit of any of claims 1 to 4 wherein the tape has two fingers that extend from one side of the anchoring portion to allow the tape to be applied in a 'Y' configuration.

7. The kit of any preceding claim wherein the patch is supplied in a kit with instructions for applying the patch to a contusion, the instructions directing a user to:
i. separate the release liner along the scored junction between the anchoring portion and the fingers,
ii. remove the release liner from the anchoring portion of the tape to expose the adhesive backing,
iii. apply the exposed adhesive backing of the anchoring portion to a contusion so that the tape adheres to the user's skin, and
iv. remove the release liner from each of the fingers and apply the exposed adhesive backing of each finger to the user's skin

8. The kit of claim 7 wherein the instructions direct the user to stretch each finger after removing the release liner and secure the respective finger in place while stretched by pressing the exposed adhesive backing against the user's skin.

9. The kit of claim 7 or 8 wherein the instructions direct the user to secure the anchoring portion of the tape to the user's skin without any tension in the anchoring portion.

10. The kit of claim 7, 8 or 9 wherein the kit includes a plurality of contusion patches connected end-to-end, the patches being folded flat with each successive patch resting on top of a previous patch, adjacent patches being connected by the frangible release liner.

11. The kit of claim 10 wherein the frangible release liner is scored between adjacent patches.

12. The kit of any preceding claim wherein the therapeutic tape is coated with a dermatologically-acceptable acrylic adhesive to create the adhesive backing.

13. A contusion patch kit comprising:
a high stretch therapeutic tape with an adhesive backing, the tape being functionally divided into sections including an anchoring portion that adheres to a contusion and a plurality of fingers that extend from the anchoring portion,
a frangible release liner covering the adhesive backing of the tape, the release liner having a greater surface area than the tape so that an exposed strip of the release liner extends around the tape's perimeter, the release liner being scored at junctions between adjacent sections of the tape to allow selective removal of the release liner during staggered application of the tape, the release liner being marked with indicators that dictate the order in which each section of the tape is to be applied, and
instructions for applying the contusion patch to a nonspecific part of the user's body using any orientation of the fingers in the user's discretion, the instructions referencing the release liner indicators and directing a user to:
i. separate the release liner along the scored junction between adjacent sections of the tape,
ii. remove the release liner from a particular section of the tape indentified by one of the indicators to expose the adhesive backing of that section,
iii. apply the exposed adhesive backing of the section to a contusion so that the tape adheres to the user's skin, and
iv. remove the release liner from each of the other sections in an order dictated by the indicators marked on the release liner and apply the exposed adhesive backing of each section in turn to the user's skin

14. The kit of claim 13 wherein the tape is less than about 5 inches in length and about 2 inches in width when unstretched, and/or wherein the instructions direct the user to secure the tape to the contusion without any tension in the tape, and/or wherein the therapeutic tape has a stretch ratio of about 1.4 or greater, and/or wherein the indicators marked on the release liner are numerals that indicate the order in which sections of the patch are to be applied.

15. The kit of claim 13 or 14 wherein the kit includes a plurality of contusion patches connected end-to-end, the patches being folded flat with each successive patch resting on top of a previous patch, adjacent patches being connected by the frangible release liner, preferably wherein the frangible release liner is scored between adjacent patches.
